(19) Europäisches Patentamt
European Patent Office
Office européen des brevets

(11) EP 4 755 312 A1

(12) EUROPEAN PATENT APPLICATION

(43) Date of publication:
10.06.2026 Bulletin 2026/24

(21) Application number: 24217064.5

(22) Date of filing: 03.12.2024

(51) International Patent Classification (IPC):
A61B 8/08 (2006.01)    A61B 8/00 (2006.01)

(52) Cooperative Patent Classification (CPC):
A61B 8/0883; A61B 8/0866; A61B 8/4245;
A61B 8/5223

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Koninklijke Philips N.V.
5656 AE Eindhoven (NL)

(72) Inventors:
• BHUNIA, Himadri Sekhar
Eindhoven 5656 AG (NL)
• SETH, Subhendu
Eindhoven 5656 AG (NL)
• V, Manikanda Krishnan
5656AG Eindhoven (NL)
• ANURADHA, Anuradha
Eindhoven 5656 AG (NL)
• GUPTA, Madhumita
Eindhoven 5656 AG (NL)
• SANTHANA NAIDU, Navaneetha Krishnan
Eindhoven 5656 AG (NL)
• VAJINEPALLI, Pallavi
Eindhoven 5656 AG (NL)
• BANIK, Debojyoti
Eindhoven 5656 AG (NL)
• VAMSI, Pavani
Eindhoven 5656 AG (NL)

(74) Representative: Philips Intellectual Property &
Standards
High Tech Campus 52
5656 AG Eindhoven (NL)

(54) PROCESSING ULTRASOUND DATA

(57) A mechanism for processing ultrasound data comprising one or more sequences of ultrasound frames captured during ultrasound imaging of a fetus. The ultrasound frames are processed to classify each ultrasound frame as a heart frame or a non-heart frame. Any clusters of ultrasound frames meeting predetermined criteria are identified, which criteria make use of the classifications of the ultrasound frames. A quality value is produced for each cluster of ultrasound frames.

FIG. 2

**Description**

FIELD OF THE INVENTION

The present invention relates to the field of obstetric ultrasound.

BACKGROUND OF THE INVENTION

Obstetric ultrasonography plays a crucial role in prenatal care for dating the gestational age of a pregnancy, identifying pregnancy risk factors, and diagnosing fetal abnormalities.

One limitation of ultrasound imaging is that of operator dependency, often requiring the operator to be proficient in interpreting ultrasound images and understanding how to position and move ultrasound probes. This dependency on operator skill can pose challenges in settings with limited trained personnel and/or where training resources are scarce.

The International Society of Ultrasound in Obstetrics and Gynecology (ISUOG) has provided a holistic and simplified protocol for obstetric ultrasound to help less skilled personnel better assess fetal parameters. However, it is recognized that this approach may not be suitable in all cases. For instance, there is a risk of not properly visualizing cardiac activity if the fetus' heart is positioned outside the scanned area. Furthermore, the iterative nature of the provided protocol may prolong scan times, leading to increased strain on both the operator and the pregnant individual.

Alternative methods for improving guiding or assessment of fetal cardiac activity are therefore desirable.

SUMMARY OF THE INVENTION

The invention is defined by the claims.

In accordance with a proposed approach, there is provided a computer-implemented method for processing ultrasound data of a fetus. The computer-implemented method comprises: receiving the ultrasound data comprising one or more sequences of ultrasound frames representing a portion of the fetus; processing each ultrasound frame of the ultrasound data to classify each ultrasound frame as a heart frame or a non-heart frame, wherein each heart frame is an ultrasound frame that is determined to contain a representation of a heart of the fetus and each non-heart frame is an ultrasound frame that is determined to fail to contain a representation of the heart of the fetus; processing the ultrasound data to identify, if present, one or more clusters of ultrasound frames; and determining, for each cluster of ultrasound frames, a quality value responsive to one or more characteristics of each heart frame in said cluster of ultrasound frames.

For each cluster of ultrasound frames: each ultrasound frame in the cluster of ultrasound frames belongs to a same sequence of ultrasound frames; the ultrasound frames in the cluster of ultrasound frames are sequentially adjacent to one another; the cluster of ultrasound frames comprises no fewer than a first predetermined number of ultrasound frames; and the cluster of ultrasound frames comprises no more than a second predetermined number of sequentially adjacent non-heart frames

The present disclosure provides a technique for producing quality value(s) for parts of ultrasound data that are most likely to contain a representation of a heart of the fetus. This provides a useful indicator of the likelihood that the ultrasound data contains sufficient information for deriving or determining a fetal heart viability (e.g., and therefore overall fetal viability). This information is useful for assessing whether further ultrasound imaging is required, for guiding the performance of future ultrasound imaging and/or for itself defining a viability confidence measure.

The ultrasound data comprises one or more sequences of ultrasound frames. For instance, each sequence may represent a different sweep of a subject using an ultrasound probe. The ultrasound frames are processed to classify each frame as a heart frame or a non-heart frame. Clusters of heart frames are then identified. Each cluster thereby represents a part of the ultrasound data that is likely to contain diagnostically useful/relevant information for making an assessment of fetal viability. A quality value is generated for each cluster of heart frames. The quality value thereby provides an indicator of whether or not the ultrasound data is likely to contain sufficient information for deriving or determining the fetal heart viability, be that automatically or a manual determination.

In some embodiments, the quality value is responsive to a total number of heart frames and a total number of non-heart frames in the cluster. This feature recognizes that clusters containing a larger number of heart frames are more likely to contain useful information for assessing cardiac activity/health of the fetus.

In some embodiments, processing each ultrasound frame of the ultrasound sound data comprises classifying each heart frame as one of a plurality of different types of heart frame, wherein the type of heart frame defines one of the one or more characteristics of the heart frame. This approach recognizes that some types of heart frame (e.g., axial heart frames) are more useful for determining cardiac activity of the fetus, e.g., contain more relevant information for diagnosing the fetal heart, compared to other types of heart frame (e.g., partial heart frames).

In some embodiments, for each cluster of ultrasound frames, the quality value of the cluster is responsive to a number of heart frames of each of the plurality of types in the cluster. By considering the number of each type of heart frame within a cluster, the quality value provides a more precise indication of the cluster's potential diagnostic value. This relies upon the above-identified recognition that some types of heart frame (e.g., axial heart frames) are more useful than other types for determining cardiac activity of the fetus.

In some embodiments, the plurality of different types of heart frame include: an axial heart frame, an off-axial heart frame, or a partial heart frame. By specifically categorizing heart frames into axial, off-axial, and partial types, the method enables information on fetal heart orientation and visibility to influence the quality value. This recognizes that the view and orientation of the fetal heart in an ultrasound frame influence or affect the diagnostic value of the ultrasound frames.

In some embodiments, processing each ultrasound frame of the ultrasound data comprises, for each heart frame, determining a measure of confidence of the classification of the heart frame; and for each cluster of ultrasound frames, the quality value of the cluster is responsive to the measure of confidence of the classification of the heart frame.

This feature incorporates a confidence measure into the quality value generation. By factoring confidence levels into the cluster quality assessment, the proposed approach is able to indicate a higher likely quality value for clusters with frames having more reliably classified heart frames. This leads to a more accurate assessment of cluster quality, prioritizes clusters with more reliable data or classifications, and reduces the impact of misclassifications.

In some embodiments, for each cluster of ultrasound frames, the quality value of the cluster is responsive to, for each type of heart frame, the mean of all measures of confidence for the classification for all heart frames in said cluster that are classified as said type of heart frame. This approach provides a more robust quality assessment of each frame. In particular, the quality value for the cluster will indicate whether the cluster contains consistently high-confidence classifications, increasing a likelihood that a cluster with a high quality value contains diagnostically relevant data.

In some embodiments, for each cluster of ultrasound frames, the quality value of the cluster is responsive to, for each type of heart frame, the standard deviation of all measures of confidence for the classification for all heart frames in said cluster that are classified as said type of heart frame. This embodiment results in the quality value indicating a consistency of classification confidence, leading to a more reliable indicator of potential diagnostic usefulness of the cluster of ultrasound frames.

In some embodiments, the method further comprises, for each cluster of ultrasound frames, processing at least the heart frames of the cluster using a machine-learning method to generate, as a viability confidence measure, a measure of confidence that the cluster of ultrasound frames indicates fetal viability, wherein, for each cluster of ultrasound frames, the quality value is responsive to the measure of confidence that the cluster of ultrasound frames indicates fetal viability.

This approach introduces an automated assessment of fetal viability into the generation of the quality value. In particular, the integration of viability assessment into the quality value calculation enables each quality value to provide a more accurate or nuanced indicator of whether or not the cluster of ultrasound frames is likely to provide meaningful insights into fetal health.

Moreover, in such approaches, the quality value itself provides an indicator of fetal viability. This provides useful diagnostic information for a clinician to support or challenge a decision on fetal viability - thereby reducing a likelihood that the clinician will make an inaccurate clinical decision.

In some embodiments, for each cluster of ultrasound frames, processing at least the heart frames of the cluster comprises extracting one or more features from the heart frames of the cluster and processing the extracted one or more features using the machine-learning method. This feature enables more sophisticated analysis by extracting relevant features from heart frames before applying machine learning. By focusing on specific features, the method is able to improve the accuracy and efficiency of fetal viability assessment.

In some embodiments, for each cluster of ultrasound frames, the extracted one or more features comprises: a histogram of oriented gradients of each heart frame of the cluster; a histogram of optical flow within the heart frames of the cluster of ultrasound frames; and/or a histogram of optical flow gradients within the heart frames of the cluster of ultrasound frames. These features have been identified as being particularly useful in the generation of an accurate viability confidence measure.

In some embodiments, the method further comprises, responsive to quality value failing to reach a predetermined threshold, generating a recommendation to perform a guided sweep. This feature introduces a dynamic sweep recommendation to an operator of the ultrasound system, e.g., indicating when further capture of ultrasound data is recommended, e.g., due to a prediction that the available ultrasound data does not comprise sufficient information to achieve high accuracy fetal viability assessment.

In some embodiments, each cluster of ultrasound frames is associated with a sweep trajectory representing a trajectory of an ultrasound imaging device during capture of the cluster of ultrasound frames; and the computer-implemented method further comprises: identifying one or more clusters of ultrasound frames responsive to the quality value of each cluster of ultrasound frames; and generating a recommended sweep trajectory responsive to the sweep trajectory associated with each identified one or more clusters.

This approach exploits information about successful or partially successful ultrasound sweeps to guide future examinations. By recommending optimal sweep trajectories based on high-quality clusters, the proposed approach is able to improve the likelihood that future scans will capture clinically relevant information about the fetal heart, thereby improving an efficiency and effectiveness of subsequent ultrasound scans and leading to more consistent and reliable fetal heart imaging.

In accordance with another proposed approach, there is provided a computer program product having computer program code means which, when executed on a computing device having a processing system, cause the processing system to perform all of the steps of the method according to any one of the previously described embodiments.

In accordance with yet another proposed approach, there is provided a processing system for processing ultrasound data, the processing system being configured to: receive the ultrasound data comprising one or more sequences of ultrasound frames representing a portion of the fetus; process each ultrasound frame of the ultrasound data to classify each ultrasound frame as a heart frame or a non-heart frame, wherein each heart frame is an ultrasound frame that is determined to contain a representation of a heart of the fetus and each non-heart frame is an ultrasound frame that is determined to fail to contain a representation of the heart of the fetus; process the ultrasound data to identify, if present, one or more clusters of ultrasound frames, wherein for each cluster of ultrasound frames: each ultrasound frame in the cluster of ultrasound frames belongs to a same sequence of ultrasound frames; the ultrasound frames in the cluster of ultrasound frames are sequentially adjacent to one another; the cluster of ultrasound frames comprises no fewer than a first predetermined number of ultrasound frames; and the cluster of ultrasound frames comprises no more than a second predetermined number of sequentially adjacent non-heart frames; determine, for each cluster of ultrasound frames, a quality value responsive to one or more characteristics of each heart frame in said cluster of ultrasound frames.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates an ultrasound system for performing a proposed method;
Fig. 2 is a flowchart illustrating a proposed method;
Fig. 3 illustrates ultrasound data;
Fig. 4 is a flowchart illustrating a proposed method;
Fig. 5 is a flowchart illustrating a proposed method;
Fig. 6 is a flowchart illustrating a proposed method; and
Fig. 7 is a flowchart illustrating a proposed method.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for processing ultrasound data comprising one or more sequences of ultrasound frames captured during ultrasound imaging of a fetus. The ultrasound frames are processed to classify each ultrasound frame as a heart frame or a non-heart frame. Any clusters of ultrasound frames meeting predetermined criteria are identified, which criteria make use of the classifications of the ultrasound frames. A quality value is produced for each cluster of ultrasound frames.

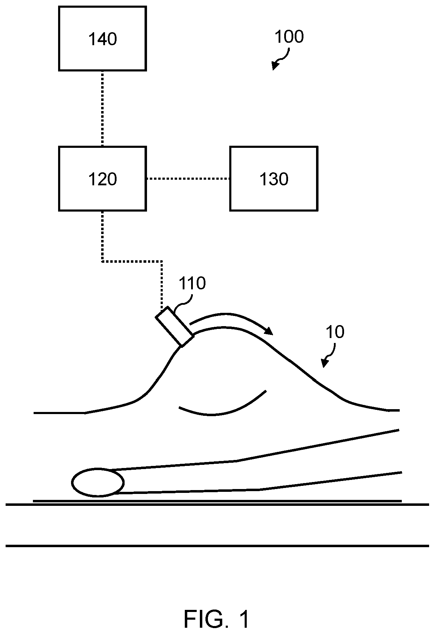

Fig. 1 shows an example of an ultrasound system 100 in which herein proposed approaches may be applied. Specifically, the ultrasound system 100 is capable of acquiring ultrasound data from a subject 10 and processing said ultrasound data according to the proposed approaches.

In the present context, the subject 10 is a pregnant female carrying a fetus (i.e., unborn child) in their uterus.

The ultrasound system 100 comprises an ultrasound probe 110 for acquiring ultrasound data from the subject. More specifically, the ultrasound probe 110 is an ultrasound transducer configured to produce ultrasound waves (which are directed into a subject) and measure reflected ultrasound waves (e.g., as reflected at tissue boundaries within the subject).

In the present context, during an ultrasound scan, the ultrasound probe 110 is placed against the abdomen of the subject 10 for imaging of the fetus. The ultrasound probe 110 may produce an (e.g., two-dimensional) fan of ultrasound waves, each of which is projected (i.e., emitted) into the subject 10 along an imaging plane. A portion of each of the ultrasound waves is reflected at each tissue boundary encountered along the imaging plane(s). In particular, said reflected ultrasound waves are reflected back towards the ultrasound probe 110 where they are subsequently measured.

By processing the intensity (i.e., amplitude) and travel time of each of the measured (reflected) ultrasound waves, an ultrasound frame (e.g., two-dimensional image data) can be produced representing a slice (i.e., a cross-section) through the subject 10. The ultrasound frame may, for instance, represent a two-dimensional slice (e.g., when the probe comprises a linear array of ultrasound sensing elements) or a three-dimensional slice (e.g., when the probe comprises an 2D array of ultrasound sensing elements). Particularly, the slice corresponds to the imaging plane(s) over which the ultrasound waves were projected over. Accordingly, by appropriately placing the ultrasound probe 110 on the subject 10, an ultrasound frame comprising a representation of the fetus (e.g., a cross-section of the fetus) can be acquired.

To provide further context, the previously mentioned travel time refers to the time interval between the ultrasound probe 110 emitting a wave of ultrasound and measuring/receiving a reflected wave of ultrasound. Assuming a constant speed of sound, the travel time for a reflected ultrasound wave is thus indicative of a depth (below the surface of the subject's abdomen) of a tissue boundary from which the ultrasound wave was reflected. Additionally, the amplitude of the measured ultrasound wave corresponds to the proportion of the initial ultrasound wave that was reflected by the tissue boundary. Accordingly, the ultrasound frame can be produced by populating pixels of a dataset based on the measured ultrasound waves, e.g., for each measured ultrasound wave, populating a specific pixel (based on the travel time of the wave and a detector channel of the ultrasound probe 110 that measured the wave) with an intensity/value proportional to the amplitude of the measured wave. This method of ultrasound imaging is better known as brightness-mode (i.e., B-mode) imaging.

During the ultrasound scan, the ultrasound probe 110 may also be moved relative to the fetus (i.e., along the surface of the subject's abdomen) while continuously emitting (and measuring) ultrasound waves. More particularly, the ultrasound probe 110 may be swept (i.e., translated) along a trajectory (i.e., path) on the surface of the subject's abdomen. A series/sequence of ultrasound frames may therefore be acquired along this trajectory, where each frame in the sequence may correspond to a respective position of the ultrasound probe 110 along the trajectory and, thus, a respective slice through the subject/fetus. Accordingly, the sequence of acquired ultrasound frames may be comprised in ultrasound data.

In this context, ultrasound data therefore comprises one or more sequences of datasets (i.e., one or more sequences of ultrasound frames). When each ultrasound frame is acquired at a respective position along an ultrasound trajectory, the ultrasound data may represent (or at least be associated with) a volume within the subject/fetus, as formed from a combination of slices.

More generally, however, the position relationship between ultrasound frames may not be linear. To be more specific, the distance (along the sweep trajectory) between consecutive ultrasound frames may not be equal, e.g., dependent on how the speed of the ultrasound probe 110 varies while acquiring ultrasound frames over the sweep trajectory. Furthermore, some ultrasound frames may be acquired from a same position along the sweep trajectory, e.g., if the ultrasound probe 110 is held still while acquiring ultrasound frames. Thus, each sequence of ultrasound frames in the ultrasound data is perhaps more precisely described by a time relationship rather than a spatial/positional relationship. Accordingly, in its raw form, each sequence may be functionally representative of a distorted volume. That being said, each sequence of ultrasound data may be converted/transformed into a true volume through an appropriate transformation, e.g., transforming the time-axis of the ultrasound data into a spatial-axis.

It will be understood that, in general, the ultrasound probe 110 is handled/maneuvered by a user (i.e., an operator) of the ultrasound system 100. A sweep trajectory traced by the ultrasound probe 110 may thus correspond to a manual trajectory controlled by the user, e.g., as opposed to a predetermined trajectory controlled by a computerized and/or robotic system. In this regard, while performing a sweep trajectory it may be useful to track movement/motion of the ultrasound probe 110 through three-dimensional space and/or relative to the subject 10. In this way, a (precise) description of the performed sweep trajectory can be known to the user and/or a computer system following acquisition of the associated ultrasound data.

In some instances, the ultrasound probe 110 may comprise a tracking system for acquiring tracking data indicative of motion of the ultrasound probe through three-dimensional space. For example, the tracking system may be an inertial navigation system (INS) comprising one or more accelerometers and/or gyroscopes for tracking/recording the position and/or orientation of the ultrasound probe 110. Accordingly, a path that the ultrasound probe traces through three-dimensional space when performing a sweep trajectory can be determined from the acquiring tracking data.

The ultrasound system 10 may additionally or alternatively comprise one or more optical tracking devices, e.g., cameras, (not visible in Fig. 1) for tracking/observing motion of the ultrasound probe relative to other objects, e.g., the subject. For example, the one or more optical tracking devices may acquire video data that can be processed, e.g., using one or more object recognition algorithms, to determine a position of the ultrasound probe 110 relative to the subject 10 at different points in time. Thus, the position and orientation of a performed sweep trajectory relative to the subject 10 (i.e., the path traced by the ultrasound probe 110 over the subject's abdomen) can be determined.

The ultrasound system 10 further comprises a processing system 120 coupled to (i.e., in communication with) the ultrasound probe 110. The processing system 120 is configured to receive or derive the ultrasound data from the ultrasound probe 110.

More precisely, the processing system 120 may be configured to receive electrical signals from the ultrasound probe 110 indicative of the ultrasound data, which the processing system 120 may then process to produce the ultrasound data. In particular, the reflected ultrasound waves measured by the ultrasound probe 110 may be converted by the ultrasound probe 110 into electrical signals indicative of an amplitude and travel time of the measured ultrasound waves.

Accordingly, the processing system 120 may process each received electrical signal to produce the ultrasound data. In particular, the processing system 120 may receive the electrical signals as a (semi-)continuous stream which the processing system 120 continually processes to produce the sequence(s) of ultrasound frames, forming the ultrasound data.

The processing system is further configured to process the received/produced ultrasound data according to a proposed method, as will be later disclosed.

The ultrasound system 100 may further comprise a memory 130 for storing data. For example, following production of ultrasound data, the processing system 120 may store the ultrasound data in the memory 130. Accordingly, the processing system 120 may later retrieve the ultrasound data from the memory 130, e.g., for use in one or more steps of a proposed method.

The ultrasound system 100 may also comprise a user interface 140. A user may interact with the user interface 140 (or more precisely with one or more interface devices comprised in the user interface 140, e.g., a keyboard) to control one or more aspects (e.g., settings) of the ultrasound system 100. The user interface 140 may also comprise a display (e.g., a screen) for displaying/presenting relevant information to the user. For example, the display may present a visualization of ultrasound data or an ultrasound frame to the user, and/or may present instructions or recommendations to the user, e.g., in relation to the later disclosure.

The present disclosure proposes a number of methods, which are preferably computer-implemented methods that may be employed by a processing system, such as the processing system 120 of the ultrasound system 100. Thus, there is also proposed a processing system configured to perform any hereafter described method, and an ultrasound system comprising the processing system 120 and any other component of the above-described ultrasound system (e.g., the ultrasound probe 110, the memory 130 and/or the user interface 140).

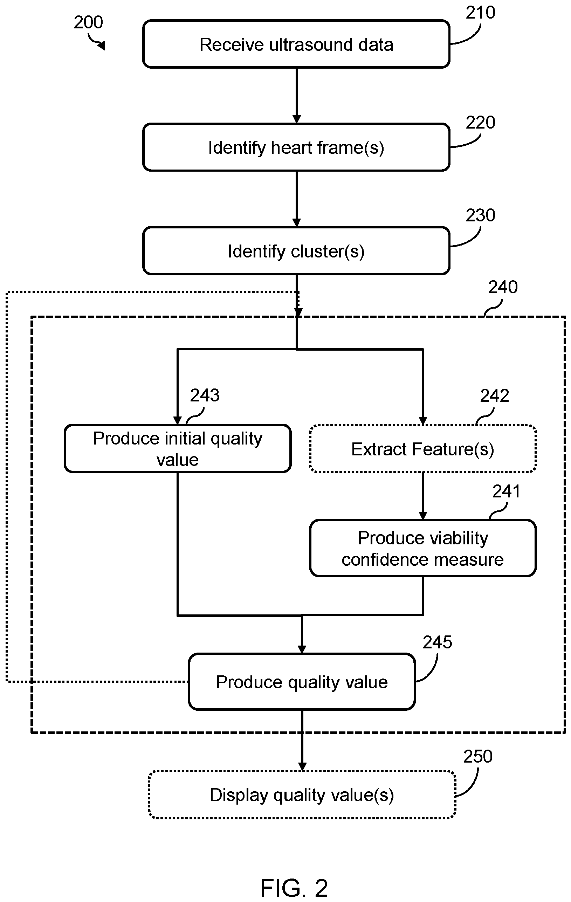

Fig. 2 is a flowchart illustrating a proposed method 200 for processing ultrasound data. The method 200 is preferably a computer-implemented method as performed by a processing system of a computing device, e.g., the processing system 120 of the ultrasound system (Fig. 1).

The method 200 comprises a step 210 of receiving the ultrasound data comprising one or more sequences of ultrasound frames representing a portion of the fetus. As previously explained, each ultrasound frame may comprise two-dimensional or three-dimensional image data.

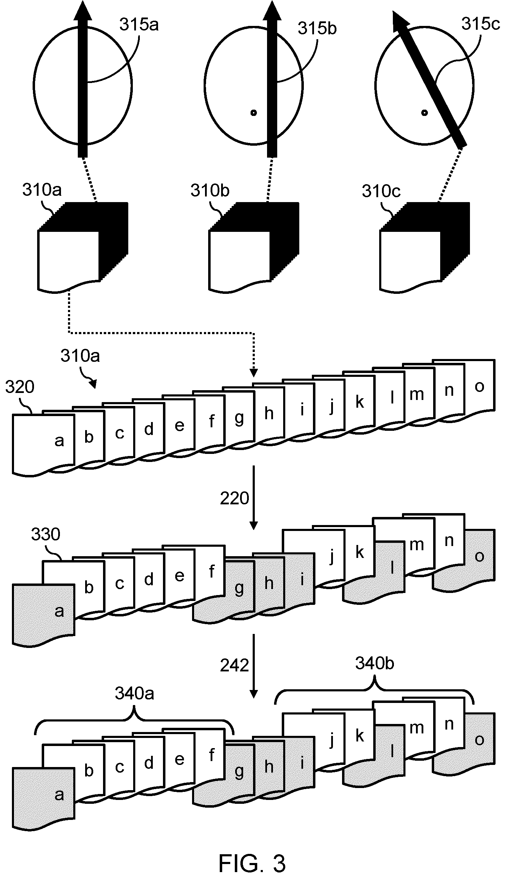

Fig. 3 illustrates examples of sequences 310a, 310b, 310c of ultrasound frames that form exemplary ultrasound data. Each sequence comprises a plurality of ultrasound frames 320 (e.g., the ultrasound frames a-o for a first example sequence 310a).

The ultrasound data may be received (e.g., by the processing system 120) as live ultrasound data, i.e., data received directly from the ultrasound probe as the data is acquired/measured. In such cases, the sequences may be received as a series of sequences (i.e., receiving each sequence one at a time) corresponding to an operator performing sequential ultrasound sweeps of the subject.

In some examples, the plurality of sequences of initial ultrasound sweep data may instead be received (or more precisely retrieved) from a memory (e.g., the memory 130). In such cases, the ultrasound data may be previously captured ultrasound data, i.e., ultrasound data that was acquired at a previous/past time and stored in the memory. The sequence(s) may be retrieved from the memory in series (i.e., retrieving each instance one at a time) or retrieved all at the same time, e.g., as a single file comprising the ultrasound data.

It will be apparent that each sequence 310a, 310b, 310c of ultrasound frames may be associated with a respective sweep trajectory 315a, 315b, 315c, which represents a movement of the ultrasound probe over the abdomen of the subject (and thus relative to the fetus carried by the subject). More specifically, each sequence of ultrasound frames comprises a plurality of ultrasound frames acquired over the associated sweep trajectory. In this way, each sequence may correspond to a different imaging path through the fetus, and thus provides a different representation of the fetus.

The sweep trajectory 315a, 315b, 315c may be exploited in some variations of the proposed method, as later explained. The sweep trajectory may, for instance, be captured and/or defined by the tracking system and/or optical tracking system (if present), as previously explained.

Turning back to Fig. 2, the method 200 further comprises a step 220 of processing each ultrasound frame of the ultrasound data to classify each ultrasound frame as a heart frame or a non-heart frame.

In this context, a heart frame is an ultrasound frame that is determined/estimated to contain a representation of a portion of a heart of the fetus (e.g., a portion or part of the heart of the fetus). The heart frames of ultrasound data thus represent relevant ultrasound frames for assessing fetal cardiac activity.

Similarly, a non-heart frame is an ultrasound frame that is determined to fail to contain a representation of the heart of the fetus. Thus, non-heart frames of ultrasound data thus represent irrelevant or less relevant ultrasound frames for assessing fetal cardiac activity.

Conceptually, step 220 may be considered as comprising a process of sorting each ultrasound frame of the ultrasound data into one of two sets, where each set respectively comprises either ultrasound frames that are determined to be heart frames or ultrasound frames that are determined to not be heart frames.

To identify a particular ultrasound frame as a heart frame, step 220 may comprise processing the ultrasound frame using a classification model to predict whether or not the ultrasound frame is a heart frame. In particular, the classification model may predict whether the ultrasound frame is a heart frame or a non-heart frame.

As later explained, the classification may be configured to, if predicting that the ultrasound frame is a heart frame, predict which of a plurality of types of heart frame the ultrasound frame is.

The classification model may be configured to identify, if present, a representation of the heart of the fetus in the ultrasound frame. More precisely, step 220 may comprise determining whether or not a representation of the heart of the fetus is present in the ultrasound frame, where a positive determination results in the ultrasound frame being identified as a heart frame and a negative determination results in the ultrasound frame being identified as a non-heart frame.

The representation of the heart of the fetus may represent a cross-section through the heart of the fetus. Additionally, said representation may typically be smaller than the ultrasound frame that comprises it, e.g., cover an area no greater than 50% of the size of the ultrasound frame, or no greater than 25% of the size of the ultrasound frame, etc. In other words, identification of the representation of the heart of the fetus typically comprises identifying a part of the ultrasound frame.

Step 220 may comprise identifying whether or not each ultrasound frame is a heart frame by processing each ultrasound frame using a classification model, such as an object detection and/or segmentation model/algorithm. Such models are capable of identifying representations of a target anatomy (e.g., the heart) in image data (e.g., an ultrasound frame) based on the presence of certain shapes and/or spectral features that resemble an expected appearance for different cross-sections of the target anatomy.

Preferably, if used, the model used in step 220 may be (or form part of) a machine-learning model (e.g., a neural network, a transformer, etc.) trained to identify representations of the heart of a fetus in ultrasound frames. One suitable example of an appropriate model is the "You-only-look-once" (YOLO) model, e.g., described by Redmon, Joseph, et al. "You only look once: Unified, real-time object detection." Proceedings of the IEEE conference on computer vision and pattern recognition (2016). Other suitable examples are well known to the skilled person.

By determining which ultrasound frames of the ultrasound sweep data are estimated to comprise a representation of the heart of the fetus, each ultrasound frame can be labelled (i.e., identified) as either being a heart frame or a non-heart frame.

Fig. 3 also illustrates an exemplary performance of step 220.

Specifically, following processing the ultrasound frames of an exemplary sequence 310a of ultrasound frames according to step 220, some of the ultrasound frames 320 are identified as heart frames 330. Specifically, ultrasound frames b-f, j, k, m, and n are identified as being heart frames 330 (e.g., responsive to identifying a representation of the heart of the fetus in said ultrasound frames), whereas ultrasound frames a, g-i, l, and o are identified as being non-heart frames (e.g., responsive to failing to identify a representation of the heart of the fetus in said ultrasound frames).

Turning back to Fig. 2, the method 200 further comprises processing 230 the ultrasound data to identify, if present, one or more clusters of ultrasound frames.

The cluster of ultrasound frames cluster is intended to comprise heart frames that are captured in a same period of time, e.g., to represent a same cardiac imaging cycle, i.e., a same instance of imaging the heart of the fetus. Accordingly, heart frames may be sorted into clusters based on their relative separation. Put another way, the one or more heart frames may be grouped into one or more clusters responsive to one or more measures of contiguousness/adjacency between consecutive heart frames.

To this end, the ultrasound frames in any given cluster of ultrasound frames are configured to meet a set of criteria that define the cluster of ultrasound frames.

A first criterion of this set of criteria is that each ultrasound frame in the cluster of ultrasound frames belongs to a same sequence of ultrasound frames. In this way, each cluster will represent data captured in a single ultrasound sweep. This ensures that the ultrasound frames in a cluster are temporally and spatially related, representing a continuous imaging sequence of the fetal heart.

A second criterion of this set of criteria is that each ultrasound frame in the cluster of ultrasound frames are sequentially adjacent to one another, within the corresponding sequence. This ensures that the cluster represents a continuous segment of the ultrasound sweep, maintaining the temporal and spatial relationships between frames. Sequential adjacency helps capture the progression of cardiac motion and structural changes within a single imaging sequence.

A third criterion of this set of criteria is that the cluster of ultrasound frames comprises no fewer than a first predetermined number of ultrasound frames. This criterion helps ensure that each cluster contains a sufficient number of frames that is likely to capture relevant information about a cardiac cycle of the fetus.

Preferably, the first predetermined number of ultrasound frames is no less than 7, e.g., no less than 15.

In brief, it is expected that a heart cycle of a fetal heart will take around 7-10 frames to complete, assuming a fetal heart rate of 120 bpm to 180 bpm and a frame capture rate of around 20 Hz (a standard assumption for ultrasound monitoring). By setting the first predetermined number of ultrasound frames to be no less than 7, the method is able to increase the likelihood that each cluster contains at least one complete cardiac cycle.

It has also been identified that, assuming a conventional movement speed of an ultrasound probe, traversal of a fetal heart (ranging from between 12-38 weeks of gestation) at a frame capture rate of 20 Hz will take around 15 frames to complete. This has been experimentally verified. By setting the predetermined number of ultrasound frames to be no less than 15, there is an increased likelihood that at least one cluster will capture an entire sweep (or a majority of a sweep) of the heart. This improves the chances of obtaining diagnostically useful information about the fetal heart structure and function. This approach also helps to ensure, for a later described embodiment, that there is sufficient data for guiding a future sweep of the subject (if performed).

A fourth criterion of this set of criteria is that the cluster of ultrasound frames comprises no more than a second predetermined number of sequentially adjacent non-heart frames. Thus, the maximum number of non-heart frames that are permitted to be adjacent to one another, whilst allowing the fourth criterion to be met, is the second predetermined number. In this way, the cluster of ultrasound frames may include a limited number of non-heart frames interspersed between heart frames, as long as no more than the second predetermined number of non-heart frames appear consecutively.

By way of example only, if the second predetermined number is set to 2, a cluster could include sub-sequences like "HHHNHH" (where H represents a heart frame and N a non-heart frame) and meet the fourth predetermined criteria, but not "HHNNNH". This approach helps maintain the integrity of each cluster as a representation of a continuous cardiac imaging sequence while allowing for minor interruptions or imperfections in the data.

This criterion helps ensure that the cluster remains focused on the heart frames while allowing for some flexibility in case of occasional non-heart frames. This may account for potential misclassifications of any ultrasound frames or brief moments where the heart is temporally not visible in the ultrasound frame(s) due to fetal movement, probe positioning and/or errors in capturing the ultrasound frame.

The second predetermined number may be non-zero to facilitate this flexibility for non-frames. In some examples, the second predetermined number is no greater than 3, e.g., no greater than 2, e.g., no greater than 1. These exemplary limits (no greater than 3, 2, or 1) aim to balance flexibility with the need to maintain the integrity and continuity of the heart frame cluster.

As a working example, with reference to Fig. 3, performing step 220 on the sequence of ultrasound data 310a may comprise first grouping ultrasound frames b-f into a first cluster 340a and ultrasound frames j-n into a second cluster 340b. Each cluster meets the set of criteria outlined above. For this working example, the first predetermined number of ultrasound frames is no greater than 5 and the second predetermined number of ultrasound frames is no greater than 2 (e.g., 1).

In some variants, the set of criteria may comprise a fifth criteria that, in the cluster of ultrasound frames, the total number of heart frames is no less than a predetermined percentage or proportion of the total number of ultrasound frames in the cluster.

This fifth criterion adds another layer of quality control to ensure that the identified clusters contain a substantial proportion of heart frames. By specifying that the total number of heart frames must be no less than a predetermined percentage or proportion of the total number of ultrasound frames in the cluster, this criterion helps to ensure that the cluster is predominantly composed of frames containing representations of the fetal heart. This approach thereby helps to filter out clusters that, while meeting the other criteria, may not contain sufficient cardiac information to be truly useful in assessing fetal viability or cardiac health.

By way of example, the predetermined percentage (or corresponding proportion) may be set to a value no less than 50%, e.g., no less than 70% or no less than 80%. Setting the predetermined percentage to a value no less than 50% ensures that the majority of frames in each cluster contain representations of the fetal heart. Higher thresholds, such as 70% or 80%, further increase the concentration of heart frames within each cluster.

The method 200 also comprises a step 240 of determining a quality value (also known as a quality score) for each of the one or more clusters identified in step 230. Specifically, each quality value for a cluster may be determined responsive to (at least) one or more characteristics for each heart frame in the cluster. Thus, conceptually, each quality value is a quality value for a subset of the ultrasound data.

In this context, the term "quality value" may refer to a numerical or categorical measure that indicates the overall suitability or usefulness of a cluster of ultrasound frames for assessing fetal cardiac activity.

A quality value is useful for comparing different clusters of ultrasound frames or to determine whether a particular cluster meets certain criteria for further analysis or clinical use. As later exemplified, a quality value may also be useful for identifying a cluster that provides the most information about a fetal cardiac activity amongst all clusters, which can be used to recommend or guide future capture of cardiac information (e.g., to match or align with a sweep trajectory used in capturing the cluster of ultrasound frames).

The one or more characteristics of a heart frame correspond to one or more features, properties, and/or parameters for (or associated with) the representation of the heart of the fetus present in the heart frame. The one or more characteristics are thus determined responsive to the representation of the heart of the fetus in each heart frame.

More specifically, the one or more characteristics for a heart frame may be associated with a perceived quality for, and/or relevance of, the representation of the heart of the fetus in the heart frame. Accordingly, the one or more characteristics may be indicative of how appropriate the heart frame (or more specifically the cross-section of the heart of the fetus associated with the heart frame) is for assessing the cardiac activity of the fetus.

In some examples, at least one of the one or more characteristics for a heart frame may comprise (or at least be indicative of) a confidence level that the heart frame contains a representation of the heart of the fetus. Specifically, the confidence level may indicate a likelihood or probability of the identified representation of the heart of the fetus actually being present in the heart frame (i.e., how confident a user or model is that the ultrasound frame actually comprises a representation of the heart of the fetus). The confidence level may thus comprise a value between 0 and 1, or a percentage between 0% and 100%, with a greater value/percentage indicating a greater likelihood of the representation of the heart of the fetus being present.

The confidence level may be sensitive to (i.e., responsive to) a perceived quality for the identified representation of the heart of the fetus, e.g., a sharpness, clearness, and/or relative contrast for the identified representation. Thus, the confidence level for a heart frame may also be indicative of how easy it would be to extract relevant information (e.g., pertaining to the cardiac activity of the fetus) from ultrasound frames acquired along the same imaging plane as that of the heart frame.

The confidence level may, for instance, be produced by the model used in step 220 to determine that the relevant heart frame is a heart frame. It is common for classification models to output a confidence level in their prediction. Thus, a confidence level may be a standard output parameter of a classification model.

In some examples, the one or more characteristics may be defined by a type of the heart frame. In particular, each heart frame may be classified as being one of a plurality of different types of heart frame, each type representing or defining a different characteristic of the heart represented in the heart frame.

Thus, for use in such embodiments, step 220 may be modified to comprise classifying each heart frame as one of a plurality of different types of heart frame, wherein the type of heart frame defines one of the one or more characteristics of the heart frame.

As an example, a type of heart frame may represent a view of the part of the heart represented in the heart frame, e.g., relative to an axis of the heart.

For instance, one type of heart frame is an axial heart frame. A heart frame may be considered to be an axial heart frame when the cross-sectional plane of the heart represented in the heart frame is perpendicular to the axis of the heart, i.e., the viewing direction of the heart frame corresponds to looking down/along the axis of the heart.

Another example of a type of heart frame is an off-axial heart frame, in which the heart frame the cross-sectional plane of the heart represented in the heart frame is not perpendicular to the axis of the heart, i.e., the viewing direction of the heart frame does not correspond to looking down/along the axis of the heart.

Another example of a type of heart frame is a partial heart frame, in which the cross-sectional plane of the heart is only partially represented in the heart frame (i.e., the representation of the cross-sectional plane of the heart is "cut off" by a boundary of the heart frame) the heart frame may be labelled as being "partial".

Thus, in some examples, a heart frame may only be considered to be an "axial" or "off-axial" heart frame when the cross-sectional plane of the heart contained within the heart frame is not cutoff by the boundary of the heart frame.

As will become relevant later, it may be preferable to image the heart of the fetus (e.g., for the purposes of assessing fetal cardiac activity) perpendicular to an axis of the heart. For example, this may provide a better cross-section for observing different vessels and/or chambers of the heart.

Thus, at least one of the one or more characteristics for a heart frame may thus comprise an index (i.e., a label) indicating a type of cross-section represented in the heart frame. More specifically, the index may be a heart frame type index indicative of a heart frame type for the heart frame, where the heart frame type corresponds to the type of cross-sectional plane of the heart of the fetus represented in the heart frame, as previously explained.

By way of example only, the heart frame type index may comprise a letter indicative of the type of represented cross-section of the heart. For example, a heart frame type index "A" may indicate an axial heart frame comprising a representation of the heart of the fetus viewed along an axis of the heart. Additionally, a heart frame type index "O" may indicate an off-axial heart frame comprising a representation of the heart of the fetus not viewed along an axis of the heart. Furthermore, a heart frame type index "P" may indicate a partial heart frame comprising a partial representation of the heart of the fetus.

As an alternative, the heart frame type index may instead comprise a number, e.g., the numbers "1", "2" and "3", where each number corresponds to a respective heart frame type.

The skilled person would be readily capable of modifying step 220 to facilitate identification of a type of heart frame. In particular, a classification model may be readily adapted to perform multi-classification (e.g., rather than binary classification). Alternatively, multiple classification models may be used, each one designed for classifying a different type of heart frame.

Thus, a binary classification model (heart frame vs. non-heart frame) could be expanded to a multi-class model that simultaneously predicts whether a frame is a non-heart frame or one of the specific heart frame types. This approach would naturally require appropriate training of the model, e.g., with a dataset that includes labeled examples of each heart frame type.

Alternatively, a two-stage classification process could be implemented. The first stage would use a binary classification model to identify whether each ultrasound frame is a heart frame or a non-heart frame. The second stage would then apply a separate multi-class model to only the identified heart frames to determine their specific type.

As another alternative, multiple binary classification models could be used, each specializing in detecting a specific heart frame type. The results from these classifiers would then be combined to determine the final classification of each frame.

Other suitable examples will be readily apparent to the appropriately skilled person.

Where a measure of confidence is used as the one or more characteristics of the heart frame, the measure of confidence for any given heart frame may be a measure of confidence that the heart frame is a measure of confidence of classification of a particular type of heart frame.

From the foregoing, it will be apparent that the one or more characteristics of a heart frame may therefore include a measure of confidence of a classification and/or a type of the heart frame (e.g., axial, off-axial and/or partial).

The quality value generated in step 240 is responsive to these one or more characteristics.

As a simple example, the quality value may simply be an average of the measures of confidence of classification. This approach provides a straightforward method to quantify the overall quality of a cluster of ultrasound frames. By averaging the confidence measures for each heart frame within the cluster, a single value is produced that represents the collective reliability of the heart frame classifications.

More sophisticated approaches for determining the quality value make use of additional factors beyond just the average confidence. Such approaches may comprise weighting different types of heart frames differently (e.g., giving more importance to axial views), determining and exploiting the distribution of confidence scores, and/or incorporating other characteristics of the heart frames as discussed earlier in the document.

As mentioned previously, it may be preferable to image the heart of the fetus perpendicular to an axis of the heart. Accordingly, the quality value for a cluster may be responsive to the types of heart frames within the cluster, or more specifically the number or proportion of each type of heart frame within the cluster. For example, a cluster comprising a greater number of axial heart frames may be assigned a greater quality value.

Equation (1) provides another example for determining the quality value S of a cluster. In this approach, $N_A$, $N_O$, and $N_P$ are the number of axial, off-axial, and partial heart frames in the cluster respectively. N is the total number of heart frames in the cluster plus the number $\Theta$ of non-heart frames encompassed by the boundaries of the cluster, i.e., $N = N_A + N_O + N_P + \Theta$. By way of example, for the second cluster 340b in Fig. 3, $\Theta = 1$. $\alpha$, $\beta$, and $\gamma$ are weighting factors. Particularly, $\alpha$, $\beta$, and $\gamma$ may follow the hierarchy $\alpha > \beta > \gamma$, corresponding to axial heart frames being the most preferred heart frame type and partial heart frames being the least preferred heart frame type.

This approach produces a basic indicator of a proportion of preferred types of heart frames in the cluster of ultrasound frames.

$$S = \alpha \frac{N_A}{N} + \beta \frac{N_O}{N} + \gamma \frac{N_P}{N} \qquad (1)$$

In more sophisticated approaches, the quality value for a cluster may be responsive to the confidence level for each heart frame in the cluster, e.g., the mean confidence level across all heart frames, or the mean confidence level for each heart frame type. For example, a cluster comprising heart frames with a greater average confidence score may be assigned a greater quality value.

Additionally and/or alternatively, the quality value may be responsive to the standard deviation of the confidence scores for the heart frames in the cluster. In particular, the quality value of the cluster may be responsive to, for each type of heart frame, the standard deviation of all measures of confidence for the classification for all heart frames in said cluster that are classified as said type of heart frame.

As a non-limiting example, the quality value S for a cluster may be determined from the following equation:

$$S = \alpha \frac{N_A}{N} \overline{C_A} \frac{e - e^{\sigma_A}}{e - 1} + \beta \frac{N_O}{N} \overline{C_O} \frac{e - e^{\sigma_O}}{e - 1} + \gamma \frac{N_P}{N} \overline{C_P} \frac{e - e^{\sigma_P}}{e - 1}, \qquad (2)$$

where $N_A$, $N_O$, and $N_P$ are the number of axial, off-axial, and partial heart frames in the cluster, respectively, $\overline{C_A}$, $\overline{C_O}$, and $\overline{C_P}$ are the means of the confidence levels for the axial, off-axial, and partial heart frames in the cluster, respectively, $\sigma_A$, $\sigma_O$, and $\sigma_P$ are the standard deviations of the confidence levels for the axial, off-axial, and partial heart frames in the cluster, respectively, $N = N_A + N_O + N_P + \Theta$ is the total number of heart frames in the cluster plus the number of non-heart frames $\Theta$ encompassed by the boundaries of the cluster (e.g., for the second cluster 340b in Fig. 3, $\Theta = 1$), and $\alpha$, $\beta$, and $\gamma$ are weighting factors.

Particularly, $\alpha$, $\beta$, and $\gamma$ may follow the hierarchy $\alpha > \beta > \gamma$, corresponding to axial heart frames being the most preferred heart frame type and partial heart frames being the least preferred heart frame type. Accordingly, a cluster comprising more axial heart frames, and heart frames with greater confidence levels (and a small confidence level variability), may be assigned a greater (and thus better) quality value S.

Furthermore, when $\alpha$, $\beta$, and $\gamma$ are properly and appropriately normalized, equation (1) may provide a normalized quality value, e.g., a quality value between 0 and 1. For example, a quality value of 1 may correspond to a "perfect" or preferred cluster comprising only axial heart frames (and not encompassing any non-heart frames) each having a confidence level of 1.

The above description thereby provides a mechanism for generating a quality value for a cluster of ultrasound frames using a measure of confidence of a classification of each heart frame in the cluster and/or a type of each heart frame (e.g., axial, off-axial and/or partial) in the cluster.

Another example approach for producing a quality value for a cluster of ultrasound frames is hereafter described, which may be exploited by the step 240.

In this approach, step 240 comprises processing 241 at least the heart frames of the cluster of ultrasound frames using a machine-learning method to generate a viability confidence measure. The viability confidence measure is a measure of confidence that the cluster of ultrasound frames indicates viability.

It will be appreciated that a viability confidence measure above a predetermined threshold indicates a viable fetus (according to the cluster of ultrasound frames). A viability confidence measure below the predetermined threshold indicates an indeterminate status for viability (i.e., rather than unviability).

A machine-learning algorithm is any self-training algorithm that processes input data in order to produce or predict output data. Here, the input data comprises sets of heart frames (and optionally all ultrasound frames in a cluster) and the output data comprises a prediction on whether or not the cluster of ultrasound frames indicates fetal viability.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

A decision tree algorithm processes input data through a tree of nodes. In the tree of nodes, each successive node splits into two or more further nodes until reaching a terminal or end node. When performing the decision tree algorithm using the tree of nodes, at each node, a decision is made as to which further node to move to next based on the input data. The end node defines the outcome of the decision tree algorithm, and therefore the machine-learning algorithm.

Methods of training a machine-learning algorithm are well known. Typically, such methods comprise obtaining a training dataset, comprising training input data entries and corresponding training output data entries.

For some machine-learning algorithms, such as a neural network, training is performed by applying an initialized machine-learning algorithm to each input data entry to generate predicted output data entries. An error between the predicted output data entries and corresponding training output data entries is used to modify the machine-learning algorithm. This process can be repeated until the error converges, and the predicted output data entries are sufficiently similar (e.g. $\pm 1\%$) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

Other approaches for training machine-learning algorithms (e.g., decision trees) are known in the art.

For instance, decision trees are often trained using a decision tree builder or learning techniques, such as those set out by Suthaharan, Shan, and Shan Suthaharan. "Decision tree learning." Machine Learning Models and Algorithms for Big Data Classification: Thinking with Examples for Effective Learning (2016): 247-269 or Ruggieri, Salvatore. "Yadt: Yet another decision tree builder." 16th IEEE International Conference on Tools with Artificial Intelligence. IEEE, 2004.

Another form of machine-learning method is a support vector machine, which can be trained by using techniques such as sequential minimal optimization or gradient descent methods. In general, support vector machines aim to find the optimal hyperplane that separates different classes in the feature space, maximizing the margin between classes. One example approach is provided by John C. Platt. "Sequential minimal optimization: a fast algorithm for training support vector machines." MSRTR: Microsoft Research 3.1 (1998): 88-95.

The training input data entries correspond to example sets of heart frames or example clusters of ultrasound frames. The training output data entries correspond to a classification of viability of the fetus representing in the corresponding heart/ultrasound frames.

The skilled person will readily appreciate how a machine-learning algorithm is able to provide or indicate a confidence measure or confidence value of its output, which here functions as the viability confidence measure.

For instance, if the machine-learning method employs a neural network, the confidence value may be derived from the activation of the output layer neuron(s). In a binary classification task, the output neuron's activation may be interpreted as a probability, with values closer to 1 indicating higher confidence in the positive class and values closer to 0 indicating higher confidence in the negative class.

For decision trees and random forests, the confidence value may be calculated as the proportion of trees that vote for a particular class. For example, if 80 out of 100 trees in a random forest predict a positive outcome, the confidence value for that prediction may be 0.8.

For support vector machines (SVMs), a confidence value may be derived from the distance between a data point and the decision boundary (hyperplane) in the feature space. For instance, in a binary classification task, the SVM may output a raw score that represents this distance. The further a data point is from the decision boundary on the positive side, the more confident the SVM is in classifying it as positive, and vice versa.

Some specific approaches for producing confidence measures for an SVM output are described by Amini, S., F. Razzazi, and K. Nayebi. "Automatic confidence measure extraction for SVM outputs using neural network." 2008 International Symposium on Telecommunications. IEEE, 2008 and/or Platt, John. "Probabilistic outputs for support vector machines and comparisons to regularized likelihood methods." Advances in large margin classifiers 10.3 (1999): 61-74 (where the output probability represents the confidence value).

Thus, in the context of assessing fetal viability from ultrasound frames, the machine learning method may output a confidence value indicating how certain it is that the input cluster of ultrasound frames indicates fetal viability.

In some approaches, processing with a machine learning method may comprise a step 242 of feature extraction followed by processing 241 (the extracted features) with a trained machine learning model to produce the viability confidence measure. Feature extraction comprises deriving one or more relevant characteristics or attributes from raw input data that represent appropriate features. This extracted feature set may then be provided as input to the trained machine learning model.

Thus, the training input data entries may instead comprise sets of one or more features extracted from the example sets of heart frames or example clusters of ultrasound frames. The training output data entries remain the same as previously described.

In some examples, the extracted feature(s) for each cluster of ultrasound frames comprises a histogram of oriented gradients (HOG) of each heart frame in said cluster or each ultrasound frame in said cluster. The HOG captures the distribution of gradient orientations in a frame or image, which is robust to lighting and pose variations.

One approach for calculating a HOG of an ultrasound frame is to compute gradients, divide into cells, create histograms, and normalize (e.g., within blocks).

The computation of cells comprises capturing the changes in intensity across the frame. This may be performed, for instance, by applying a simple 1D point discrete derivative mask in both the horizontal and vertical directions. Next, the frame is divided into called cells. For each cell, a histogram of gradient directions is then accumulated. The contribution of each pixel to the histogram may be weighted by the gradient magnitude, to configured stronger edges to have a greater influence on the final output. The histograms of the cells are then normalized, which may be on a per-frame scale or on a block-scale. This normalization step provides invariance to local illumination and contrast changes. The HOG descriptor for the entire ultrasound frame may then be formed, for instance, by concatenating these normalized cell histograms.

In some examples, the extracted feature(s) for each cluster of ultrasound frames comprises a histogram of optical flow (HOF) within the heart frames of the cluster of ultrasound frames. The HOF represents motion patterns in an image sequence, capturing movement of objects.

One approach for calculating a HOF is to compute optical flow, bin orientations into histograms, and normalize (e.g., within blocks).

By way of example, optical flow may be computed between consecutive frames to estimate the apparent motion of objects or features. This may comprise calculating the displacement vector for each pixel or region between frames.

Next, the orientations of these displacement vectors are binned into histograms, to discretize the continuous range of motion directions into a fixed number of orientation bins. This effectively creates a representation of the motion patterns. The histograms may then be normalized, preferably within spatial blocks of the ultrasound frame. The resulting HOF provides a robust representation of motion patterns.

In some examples, the extracted feature(s) for each cluster of ultrasound frames comprises a histogram of optical flow gradients (HOFG) within the heart frames of the cluster of ultrasound frames.

One approach for calculating a HOFG is to compute optical flow gradients, bin orientations into histograms, and normalize within blocks.

By way of example, computing the optical flow gradients may comprise calculating the spatial derivatives of the optical flow field, representing how motion vectors change across the ultrasound frame. Next, the orientations of these optical flow gradients are binned into histograms. This process functions to discretize the range of gradient orientations into a fixed number of bins, representing the gradient distribution. Finally, the histograms may be normalized, e.g., within spatial blocks of the ultrasound frame. The resultant HOFG represents both the motion patterns and their spatial changes within the ultrasound frames.

In some examples, the viability confidence measure itself represents or functions as the quality measure for the cluster of ultrasound frames.

In other examples, step 240 comprises (for each cluster of ultrasound frames) processing 243 a first set of one or more characteristics of a heart frame to generate an initial quality value.

The first set of one or more characteristics therefore include a measure of confidence of a classification and/or a type of the heart frame (e.g., axial, off-axial and/or partial). Any suitable approach for generating a quality value that makes use of these example characteristics may be similarly used to generate the initial quality value, such as those exemplified by Equations (2) and (3).

In such examples, step 240 comprises (for each cluster of ultrasound frames) combining 245 the viability confidence measure of the cluster with the initial quality value of the cluster to produce the quality value for the cluster. This combination may, for instance, comprise multiplying the initial quality value and the viability confidence measure together or averaging the initial quality value and the variability confidence measure together.

In other variants (e.g., where steps 241 and 242 are omitted), then the initial quality value functions as the quality value for the cluster.

It will be appreciated that step 240 is repeated for each cluster of ultrasound frames identified in step 230.

The present disclosure envisages and proposes a number of uses for the quality value of the clusters.

One example use is illustrated in Fig. 2, in which the method 200 comprises an (optional) step 250 of controlling an output user interface to provide a visual representation of (i.e., display) one or more quality values by the preceding steps.

Step 250 may comprise controlling the output user interface to provide a visual representation of all quality values determined by the preceding steps. In other examples, step 250 may comprise controlling the user interface to provide a visual representation of only the top X quality values, where a top quality value is a quality value indicating the highest quality amongst the quality values and the value of X is a non-zero integer (e.g., 1, 2 or 5).

This recognizes that the quality value assigned to each cluster of ultrasound frames provides valuable information for aiding in the assessment of fetal viability.

Specifically, the identified clusters of ultrasound frames represent portions of the ultrasound data that are most likely to contain useful information for assessing fetal viability. This is because the clusters are defined by criteria that favor contiguous sequences of heart frames, which are the most relevant for evaluating cardiac activity and, by extension, fetal viability.

The quality value further refines this selection by quantifying how well each cluster meets these criteria and how confidently the frames within the cluster represent cardiac structures. A higher quality value indicates not only a greater concentration of heart frames but may also indicate those clusters that are more likely to contain more diagnostically useful images of the fetal heart. This means that even without further processing, the quality values themselves can guide clinicians or automated systems to focus on the most promising segments of the ultrasound data for detailed analysis of fetal cardiac activity and overall viability assessment.

Similarly, the quality values can serve as a quick indicator of the overall quality of the ultrasound examination. If all identified clusters have low quality values, it may suggest that the entire ultrasound sweep was suboptimal and may need to be repeated. Conversely, the presence of clusters with high quality values can provide confidence that at least some portions of the ultrasound data are likely to yield reliable information about fetal viability.

Determining the quality value for each cluster of ultrasound frames also provides a useful indicator of whether additional imaging is likely to be necessary or warranted to produce diagnostically useful image data of the fetal heart. If there is one or more cluster with a sufficiently high quality value, that it can be assumed that diagnostically useful image data of the fetal heart has been captured (e.g., is contained in the cluster(s) having the sufficiently high quality value).

When employed, the use of a viability confidence measure in producing the quality value provides an automated and objective indication of fetal viability. By incorporating the viability confidence measure into the quality value calculation, the method provides a robust indicator of fetal viability. In this way, when a viability confidence measure is generated, the displayed quality value(s) provides an indicator of predicted fetus viability. More specifically, the largest quality value will represent an overall predicted likelihood of fetus viability. It is again emphasized that a low quality value may not necessarily mean that the fetus is unviable, rather it indicates that fetus viability is indetermined.

In particular, when combining the viability confidence measure and the initial quality score to produce the quality score for each cluster, the quality score provides takes into account both the perceived usefulness of the ultrasound frames for assessing fetal viability and the machine learning-based assessment of viability indicators present in the frames to provide an even more robust and confident indicator of fetal viability.

More particular, the combination of these factors serves to amplify the significance of high-quality clusters that also show strong indicators of fetal viability, while reducing the impact of clusters that may have high image quality but lack clear viability indicators (or vice versa). This method of combining the initial quality score with the viability confidence measure makes the final quality value more robust and reliable for clinical interpretation.

Thus, in approaches which make use of a viability confidence measure, the performance of step 250 provides an operator with an automated assessment of fetal viability based on the ultrasound data.

Step 250 may comprise responding to one or more user interactions with the displayed quality score(s), e.g., to provide a visual representation of the cluster of ultrasound frames having the associated quality score. For example, when a user selects or clicks on a displayed quality score, the system may respond by presenting the corresponding cluster of ultrasound frames on the user interface. This visual representation could include displaying the sequence of ultrasound frames within the selected cluster, potentially highlighting the heart frames or other relevant features. This approach can also aid in the interpretation of the quality scores by providing immediate visual context.

As alluded to above, the quality value of each cluster also facilitates automated ranking of the clusters, e.g., to provide an initial prioritization of the cluster of ultrasound frame(s) for later manual review. This initial prioritization can significantly streamline the analysis process, allowing medical professionals to focus their attention on the ultrasound data segments most likely to yield valuable diagnostic information about fetal cardiac activity and overall viability.

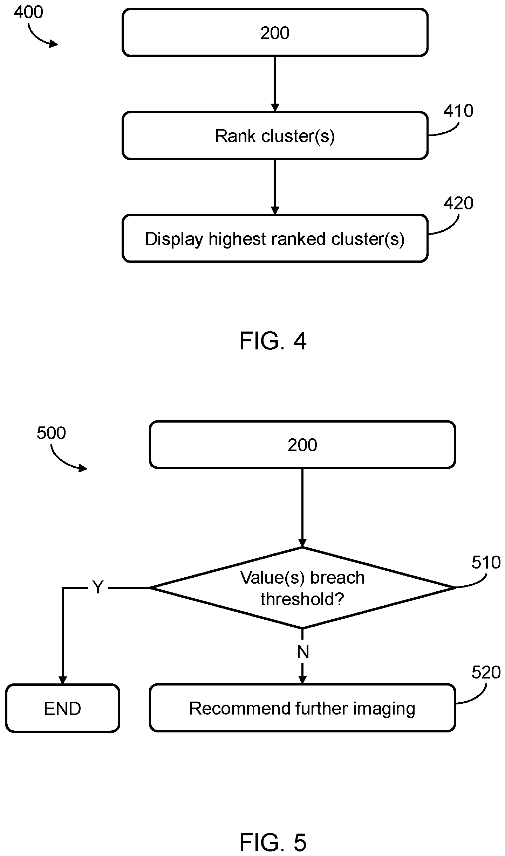

Fig. 4 illustrates a proposed method 400 according to an embodiment. The method 400 is a computer-implemented method that may be employed by a processing system, such as that previously described.

The method comprises performing the method 200 previously disclosed to produce, for one or more clusters (if present), a respective quality value.

The method 400 also comprises a step 410 of ranking or ordering the cluster(s) by their quality value, to determine an order of the clusters by their quality values.

The method 400 also comprises a step 420 of controlling an output user interface to provide a visual representation of at least the cluster having the highest quality value (i.e., the highest quality cluster). This provides an operator with a quick and efficient way to review the most promising ultrasound data for assessing fetal cardiac activity and viability. The visual representation may include displaying the ultrasound frames of the highest quality cluster in sequence, highlighting key features, or providing summary statistics about the cluster's characteristics.

Of course, step 420 may be configured to permit user interaction to select one or more different cluster(s) of ultrasound frames for display. For instance, step 420 may comprise displaying a list of the clusters of ultrasound frames, e.g., ordered by their quality value, and responding to one or more user interactions with the displayed list to provide a visual representation of any cluster identified by the user (in the user interaction(s)).

However, the quality value assigned to each cluster of ultrasound frames provides valuable information beyond simply facilitating the ranking the clusters.

Fig. 5 illustrates a proposed method 500 according to an embodiment. The method 500 is a computer-implemented method that may be employed by a processing system, such as that previously described.

The method comprises performing the method 200 previously disclosed to produce, for one or more clusters (if present), a respective quality value.

The method 500 also comprises a step 510 of comparing each quality value to a predetermined quality threshold.

Responsive to each quality value failing to breach (e.g., is less than) the predetermined threshold, the method 500 may perform step 520 of generating a recommendation that further imaging is performed. Otherwise, no recommendation is generated, and the method 500 may end.

Step 520 may comprise controlling a user interface to provide a visual representation indicating that further imaging is recommended to be performed. The visual representation may include a brief explanation of why further imaging is recommended, e.g., referencing the quality value(s) of the existing cluster(s), providing transparency about the decision-making process and helping the operator understand the importance of the additional imaging.

It will be appreciated that methods 400 and 500 may be performed simultaneously. Thus, there is proposed a method that comprises performing method 200, as well as steps 410, 420, 510 and 520.

Another use for the generated quality value(s) is to guide or recommend a sweep trajectory for further imaging of the subject.

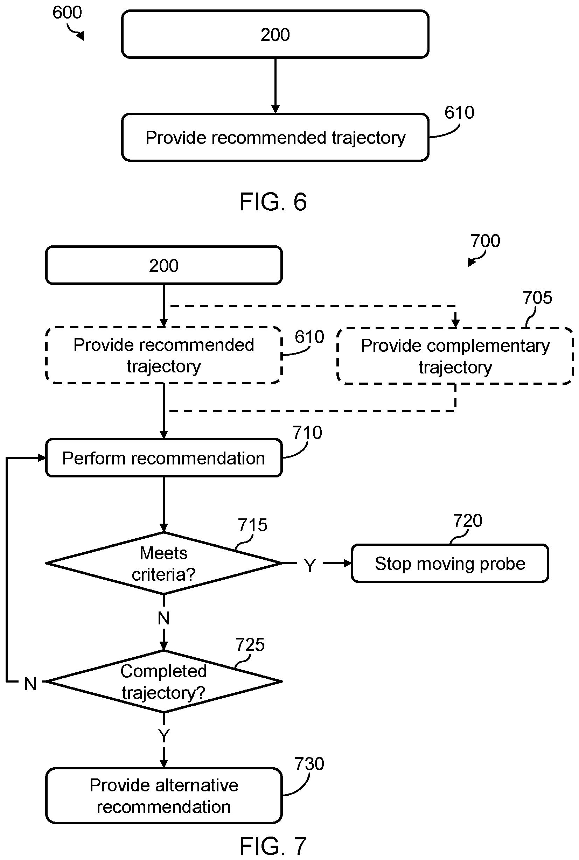

Fig. 6 illustrates a proposed method 600 according to an embodiment, which generates a recommended sweep trajectory. The method 600 is a computer-implemented method that may be employed by a processing system, such as that previously described.

The method 600 comprises performing the method 200 previously disclosed to produce, for one or more clusters (if present), a respective quality value.

The method 600 further comprises a step 610 of providing a recommended sweep trajectory, or more precisely an indication/representation of the recommended sweep trajectory.

Particularly, step 610 may comprise providing a user-perceptible output representing the recommended sweep trajectory to a user, e.g., in the form of a description, image, or graphical representation presented on a display of a user interface (e.g., the user interface 140 of Fig. 1).

Immediately prior to performing step 610, it will be understood that each cluster will have each been assigned an associated quality value. Accordingly, step 610 comprises determining the recommended sweep trajectory responsive to the quality value for each cluster of ultrasound frames.

It will also be understood that each cluster of ultrasound frames is associable with a sweep trajectory. In particular, each cluster of ultrasound frames belongs to a particular sequence, which is itself associated with a sweep trajectory. For each cluster, the sweep trajectory of its sequence may be associated to the cluster or a portion of the sweep trajectory that corresponds to part of the sequence forming the cluster may be associated to the cluster.

As a working example, step 610 may comprise recommending a sweep trajectory corresponding to the sweep trajectory for the cluster with the highest determined quality, i.e., as indicated by the quality score. In accordance with the previous disclosure, the cluster with the highest determined quality may correspond to the cluster with the greatest quality score, e.g., when the quality score is determined from one or more cluster scores. As an alternative, however, the cluster with the highest determined quality may correspond to the cluster with the lowest quality score, e.g., when the quality score is representative of a degree of artifacts (or other negative image features) present in the cluster.

Particularly, according to the previous disclosure, the recommended sweep trajectory may correspond to a preferred/appropriate sweep trajectory for imaging the heart of the fetus (e.g., that results in the heart being preferably imaged along a desired axis and/or with a desired level of image quality). Thus, the recommended sweep trajectory may comprise a preferred/appropriate sweep trajectory for assessing fetal cardiac activity.

When a representation of the recommended sweep trajectory is known (e.g., from tracking data and/or video data acquired during acquisition of the associated ultrasound sweep data), step 610 may comprise displaying said representation on the display of the user interface. Particularly, the recommended sweep trajectory may be displayed as a path relative to a representation of an abdomen of a subject (e.g., a representation of the present subject's abdomen).

Alternatively, when a representation of the recommended sweep trajectory is unknown, the recommended sweep trajectory may be provided as a label, index, description, or message indicative of the recommended sweep trajectory. For example, when the recommended sweep trajectory is the sweep trajectory associated with, e.g., a cluster found in a fourth sequence amongst a plurality of sequences, step 610 may comprise providing a message recommending the trajectory of the fourth sequence. The user may then recall/remember the sweep trajectory they performed when acquiring the fourth sequence.

In the example method illustrated by Fig. 6, the generation of the recommended sweep trajectory is always performed after completion of method 200. However, in alternative examples, a recommended sweep is only generated responsive to the quality value breaching the predetermined threshold. Thus, referring also to Fig. 5, step 520 may be configured to further comprise performing step 610.

This conditional approach to generating sweep trajectory recommendations can potentially save time and resources in ultrasound examinations. It allows the system to differentiate between cases where additional imaging might yield better results and those where the existing data is already adequate for assessing fetal cardiac activity and viability.

Fig. 7 is a flowchart illustrating a proposed method 700. Specifically, the method 700 comprises all of the steps of the method 200 and step 610 together with a number of further (optional) steps.

The skilled person would appreciate that, in some examples, the steps 610, and the following optional steps illustrated by Fig. 7, are only performed responsive to the quality value breaching the predetermined threshold. Thus, referring also to Fig. 5, step 520 may be configured to further comprise performing step 610 and the other optional steps illustrated by Fig. 7.

In certain cases, an operator wishing to assess the cardiac activity of a fetus may wish to observe the workings and/or motion of the heart of the fetus, e.g., to assess heart development. Particularly, the operator may wish to acquire a series of images of the heart at a fixed cross-section (i.e., acquire a series of ultrasound frames from a fixed imaging plane) over one or more cardiac cycles. This can be achieved by holding an ultrasound probe in an appropriate fixed position on the subject's abdomen for a sufficient period of time while acquiring ultrasound frames.

The further steps of the method 700 may assist the operator in appropriately positioning the ultrasound probe to achieve this goal.

In particular, the method 700 may comprise a step 705 of providing (an indication/representation of) one or more complimentary sweep trajectories in addition to the recommended sweep trajectory (generated in step 610). Specifically, the one or more complimentary sweep trajectories correspond to sweep trajectories associated with the sequences of ultrasound frames (i.e., sweep trajectories performed to acquire the plurality of sequences of ultrasound frames). Furthermore, the one or more complimentary sweep trajectories correspond to sweep trajectories that intersect (i.e., cross paths with) the recommended sweep trajectory.

More particularly, the one or more complimentary sweep trajectories are sweep trajectories that have been determined (e.g., as part of step 705) to intersect the recommended sweep trajectory in the vicinity of the heart of the fetus. Accordingly, when placed on the subject's abdomen at a position corresponding to the intersection point of the recommended sweep trajectory and the one or more complimentary sweep trajectories, the ultrasound probe may acquire ultrasound frames that comprise a representation of the heart of the fetus.

The one or more complimentary sweep trajectories may be determined by (first) analyzing (the representations of) each of the sweep trajectories associated with each sequence of ultrasound frames to identify an initial set of sweep trajectories that intersect the recommended sweep trajectories. The one or more complimentary sweep trajectories may then be selected from this initial set responsive to the quality values of any cluster in the corresponding sequences.

In some specific examples, only sweep trajectories for sequences having a cluster associated with a high level of quality (e.g., a high quality value) are selected as a complimentary sweep trajectory. Under the proposed approaches for determining a quality value, a better quality value may indicate that the corresponding sweep trajectory preferably passes through/over the heart of the fetus.

In some examples, step 705 may comprise identifying clusters with quality values breaching a predetermined threshold or being among the Y highest quality values, where Y is a positive integer. These identified clusters may then be used to determine complimentary sweep trajectories.

Of course, to identify such clusters, the method may compare the quality value of each cluster to a predetermined threshold. Clusters with quality values exceeding this threshold may be selected. Alternatively, the method may rank the clusters based on their quality values and select the top Y clusters, where Y is a predetermined number.

Once the relevant clusters are identified, their corresponding sweep trajectories may be designated as complimentary sweep trajectories.

In some cases, the method may limit the number of complimentary sweep trajectories to a predetermined maximum to avoid overwhelming the operator with too many options. This maximum may be determined based on factors such as the complexity of the fetal position, the quality of the initial ultrasound data, or user preferences.

Step 705 may comprise providing the one or more complimentary sweep trajectories on the display of the operator-interface. Particularly, the one or more complimentary sweep trajectories may be displayed together with the recommended sweep trajectory, e.g., overlaid, such that an operator may observe/perceive the intersection point of the different sweep trajectories.

In some examples, step 705 is omitted.

The method 700 may comprise a step 710 of providing an indication (e.g., to the operator) to perform (and thus acquire ultrasound sweep data over) the recommended sweep trajectory. Particularly, said indication may be presented to the operator on the display of the operator-interface.

In response to the operator performing the recommended sweep trajectory (and/or the processing system receiving ultrasound sweep data/ultrasound frames), the method 700 moves to a step 715 of determined whether or not the (newly) acquired ultrasound sweep data meets one or more predefined criteria. Specifically, the one or more predefined criteria may be associated with criteria that indicate when the ultrasound probe is in an adequate position for acquiring a series of ultrasound frames from the heart of the fetus, and/or requirements for ultrasound frames acquired from the heart of the fetus.

For example, the one or more predefined criteria may comprise a criterion related to whether or not a sufficient number of consecutive heart frames have (presently) been acquired. To provide further context, as ultrasound frames are acquired during a performance of the recommended sweep trajectory, each ultrasound frame may be processed to determine whether or not is contains a representation of the heart of the fetus (i.e., whether or not it is a heart frame). Accordingly, once a sufficient number of heart frames have been measured consecutively (and thus the criterion is met) it may be inferred that the ultrasound probe is (currently) over the heart of the fetus, and thus may be in an appropriate position for assessing the fetus' cardiac activity.

The one or more predefined criteria may (also) comprise a criterion of a present heart frame (i.e., a most recently generated heart frame) comprising a representation of a desired cross-section of the heart of the fetus. Particularly, each acquired heart frame may be processed (e.g., using an image classification model) to determine whether the representation of the heart of the fetus in the heart frame corresponds to the desired cross-section (and thus satisfies the criterion).

As another example, the one or more predefined criteria may comprise a criterion related to whether or not the representation of the heart of the fetus in a present heart frame is of a sufficient image quality. Particularly, the criterion may be met if the image quality for the representation of the heart of the fetus in the present heart frame is sufficiently high.

Following a positive determination in step 715 (i.e., the one or more predefined criteria are met) the method 700 progresses to a step 720 of providing an indication to stop moving the ultrasound probe. For example, said indication may be provided to the operator through the operator-interface, e.g., in the form of a messaged presented on the display and/or an audio alert. Particularly, the indication is intended to instruct the operator to hold the ultrasound probe in place as it may be in an appropriate position for assessing the cardiac activity of the fetus (i.e., as inferred from the one or more predefined criteria being met).

Following a negative determination in step 715, however, the method 700 progresses to a step 725 of determining whether or not the recommended sweep trajectory has been completed. For instance, said determination may be made in response to tracking data acquired by the ultrasound probe, e.g., which may be indicative of whether or not the ultrasound probe has reached the end of the recommended sweep trajectory.

Following a negative determination in step 725, the method 700 returns to step 710, thus instructing the operator to continue performing the recommended sweep trajectory.

The steps 710, 715 and 725 may thus represent a process of monitoring the ultrasound sweep data acquired during a performance of the recommended sweep trajectory. Particularly, said monitoring process may be used to determine whether or not the current ultrasound frames of the ultrasound sweep data (i.e., as have been acquired up to a present point in the performance of the recommended sweep trajectory), or at least the most recent ultrasound frames of the ultrasound sweep data, meet certain criteria indicative of the ultrasound probe being in an appropriate position for assessing fetal cardiac activity.

Following a positive determination in step 725, the method 700 progresses to a step 730 of providing an alternative recommended sweep trajectory.

In the present context, a positive determination in step 725 may only be achieved if there has been no positive determination in step 715 throughout the entirety of the recommended sweep trajectory, i.e., no position along the recommended sweep trajectory has been determined to be appropriate for assessing fetal cardiac activity. This scenario may occur if the fetus moves between acquiring the (original) sequences of ultrasound frames and performing the recommended sweep trajectory, e.g., such that the heart of the fetus no longer lies along the recommended sweep trajectory.

Accordingly, step 730 provides an alternative recommended sweep trajectory (being a sweep trajectory different to the recommended sweep trajectory) along which an appropriate position for assessing fetal cardiac activity may be found.

In some examples, the alternative recommended sweep trajectory may correspond to a translation of the recommended sweep trajectory (i.e., a displacement of the recommended sweep trajectory on the subject's abdomen). Particularly, said translation may be determined by comparing the sequence of ultrasound frames associated with the recommended sweep trajectory to the sequence of ultrasound data ultrasound data acquiring following step 710. For example, said comparison may determine a correspondence between the two sequences of ultrasound data indicative of a translation between the two sequences of ultrasound data.

In other examples, the alternative recommended sweep trajectory may correspond to a further sweep trajectory associated with the sequences of ultrasound data in the ultrasound data. For example, the alternative recommended sweep trajectory may (typically) be the sweep trajectory associated with the sequence of ultrasound frames with the next best quality value, i.e., compared to the quality value for the sequence of ultrasound frames associated with the recommended sweep trajectory.

Accordingly, by performing the alternative recommended sweep trajectory, the operator may locate an appropriate position for assessing the cardiac activity of the fetus.

Turning back to Fig. 2, some further optional variants of method 200 are hereafter described.

In one variant of method 200, the method 200 is configured to exit at step 230 if no clusters are identified in the ultrasound data. This early exit condition ensures that the method does not proceed unnecessarily when there are no suitable clusters of heart frames to analyze, thereby saving processing power when there is a low likelihood that there will be suitable information for making an assessment of fetal viability.

In such cases, the method 200 may optionally generate a recommendation for the operator to perform another ultrasound sweep, e.g., control a user interface to provide a user-perceptible output to recommend the performance of a further ultrasound sweep, if no clusters are identified in the ultrasound data.

By way of example, method 200 may be configured to, responsive to no clusters being identified in the ultrasound data, perform step 610 (Fig. 6). This recommendation recognizes that the absence of identifiable clusters may indicate suboptimal imaging conditions or fetal positioning during the initial ultrasound sweep.

By recommending another ultrasound sweep, the method 200 provides an opportunity to acquire more suitable ultrasound data that may yield identifiable clusters of heart frames. This additional sweep could involve adjusting the ultrasound probe position, angle, or trajectory to better capture the fetal heart. The recommendation may be presented to the operator through the user interface, potentially including guidance on how to modify the sweep technique to improve the likelihood of capturing suitable heart frames.

In some variants, method 200 may be configured to perform step 243 for each cluster (i.e., all of the one or more clusters) before performing any instance of step 241 and 242 for any of the clusters.

Specifically, in the performance of step 240, an initial quality value is determined for each cluster using only the first set of one or more characteristics of the heart frames, without considering the viability confidence measure. This approach allows for a preliminary assessment of cluster quality based solely on image characteristics.

In some examples, if no initial quality value breaches a predetermined threshold, the method 200 may exit at step 243. This early exit condition ensures that the method does not proceed with further processing if none of the identified clusters meet a minimum quality standard, indicating that it is unlikely that any cluster of ultrasound frames will contain enough information to make a reliable prediction on fetal viability.

This approach recognizes that the processing of ultrasound frames using a machine-learning method (to produce the viability confidence measure) is a resource-intensive task. By avoiding unnecessary performance of this resource-intensive task, significant time and/or power savings can be achieved.

The predetermined threshold may be set based on empirical data or expert knowledge to represent a minimum level of image quality and heart frame consistency desired for reliable fetal cardiac assessment.

In cases where the method exits at step 243 due to no clusters meeting the quality threshold, the system may optionally generate a recommendation for the operator to perform another ultrasound sweep. This recommendation recognizes that the absence of high-quality clusters may indicate suboptimal imaging conditions or fetal positioning during the initial ultrasound sweep. By suggesting another sweep, the system provides an opportunity to acquire more suitable ultrasound data that may yield higher quality clusters of heart frames.

The recommendation for an additional ultrasound sweep may include guidance on adjusting the ultrasound probe position, angle, or trajectory to improve the likelihood of capturing suitable heart frames. This guidance may be based on the characteristics of the existing clusters, even if they did not meet the quality threshold, to suggest modifications that might lead to better imaging of the fetal heart.

By way of example, method 200 may be configured to, responsive to no clusters meeting the quality threshold, perform step 610 (Fig. 6). This recommendation recognizes that the absence of identifiable clusters may indicate suboptimal imaging conditions or fetal positioning during the initial ultrasound sweep.

By implementing this approach, the method ensures that it only proceeds with further analysis, including the computation of viability confidence measures, when there is at least one cluster of sufficient initial quality. This helps to conserve computational resources and prevents the generation of potentially unreliable assessments based on low-quality data.

Proposed embodiments make use of a processing system.

The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A computer-implemented method (200, 400) for processing ultrasound data of a fetus, the computer-implemented method comprising:

   receiving (210) the ultrasound data comprising one or more sequences (310a, 310b, 310c) of ultrasound frames (320) representing a portion of the fetus;
   processing (220) each ultrasound frame (a - o) of the ultrasound data to classify each ultrasound frame as a heart frame (330) or a non-heart frame, wherein each heart frame is an ultrasound frame that is determined to contain a representation of a heart of the fetus and each non-heart frame is an ultrasound frame that is determined to fail to contain a representation of the heart of the fetus;
   processing (230) the ultrasound data to identify, if present, one or more clusters (340a, 340b) of ultrasound frames, wherein for each cluster of ultrasound frames:

      each ultrasound frame in the cluster of ultrasound frames belongs to a same sequence of ultrasound frames;
      the ultrasound frames in the cluster of ultrasound frames are sequentially adjacent to one another;
      the cluster of ultrasound frames comprises no fewer than a first predetermined number of ultrasound frames; and
      the cluster of ultrasound frames comprises no more than a second predetermined number of sequentially adjacent non-heart frames; and

   determining (240), for each cluster of ultrasound frames, a quality value responsive to one or more characteristics of each heart frame in said cluster of ultrasound frames.

2. The computer-implemented method of claim 1, wherein the quality value is responsive to a total number of heart frames and a total number of non-heart frames in the cluster.

3. The computer-implemented method of any one of claims 1 or 2, wherein processing each ultrasound frame of the ultrasound sound data comprises classifying each heart frame as one of a plurality of different types of heart frame, wherein the type of heart frame defines one of the one or more characteristics of the heart frame.

4. The computer-implemented method of claim 3, wherein, for each cluster of ultrasound frames, the quality value of the cluster is responsive to a number of heart frames of each of the plurality of types in the cluster.

5. The computer-implemented method of claim 3 or 4, wherein the plurality of different types of heart frame include: an axial heart frame, an off-axial heart frame, or a partial heart frame.

6. The computer-implemented method of any one of claims 3 to 5, wherein:

   processing each ultrasound frame of the ultrasound data comprises, for each heart frame, determining a measure of confidence of the classification of the heart frame; and
   for each cluster of ultrasound frames, the quality value of the cluster is responsive to the measure of confidence of the classification of the heart frame.

7. The computer-implemented method of claim 6, wherein, for each cluster of ultrasound frames, the quality value of the cluster is responsive to, for each type of heart frame, the mean of all measures of confidence for the classification for all heart frames in said cluster that are classified as said type of heart frame.

8. The computer-implemented method of claim 6 or 7, wherein, for each cluster of ultrasound frames, the quality value of the cluster is responsive to, for each type of heart frame, the standard deviation of all measures of confidence for the classification for all heart frames in said cluster that are classified as said type of heart frame.

9. The computer-implemented method of any one of claims 1 to 8, further comprising, for each cluster of ultrasound frames, processing at least the heart frames of the cluster using a machine-learning method to generate, as a viability confidence measure, a measure of confidence that the cluster of ultrasound frames indicates fetal viability, wherein, for each cluster of ultrasound frames, the quality value is responsive to the measure of confidence that the cluster of ultrasound frames indicates fetal viability.

10. The computer-implemented method of claim 9, wherein, for each cluster of ultrasound frames, processing at least the heart frames of the cluster comprises extracting one or more features from the heart frames of the cluster and processing the extracted one or more features using the machine-learning method

11. The computer-implemented method of claim 10, wherein for each cluster of ultrasound frames, the extracted one or more features comprises:

a histogram of oriented gradients of each heart frame of the cluster;
a histogram of optical flow within the heart frames of the cluster of ultrasound frames; and/or
a histogram of optical flow gradients within the heart frames of the cluster of ultrasound frames.

12. The computer-implemented method of any one of claims 1 to 11, further comprising, responsive to quality value failing to reach a predetermined threshold, generating a recommendation to perform a guided sweep.

13. The computer-implemented method of any one of claims 1 to 12, wherein:

each cluster of ultrasound frames is associated with a sweep trajectory representing a trajectory of an ultrasound imaging device during capture of the cluster of ultrasound frames; and
the computer-implemented method further comprises:

identifying one or more clusters of ultrasound frames responsive to the quality value of each cluster of ultrasound frames; and
generating a recommended sweep trajectory responsive to the sweep trajectory associated with each identified one or more clusters.

14. A computer program product having computer program code means which, when executed on a computing device having a processing system (120), cause the processing system to perform all of the steps of the method according to any one of claims 1 to 13.

15. A processing system (120) for processing ultrasound data, the processing system being configured to:

receive the ultrasound data comprising one or more sequences of ultrasound frames representing a portion of the fetus;
process each ultrasound frame of the ultrasound data to classify each ultrasound frame as a heart frame or a non-heart frame, wherein each heart frame is an ultrasound frame that is determined to contain a representation of a heart of the fetus and each non-heart frame is an ultrasound frame that is determined to fail to contain a representation of the heart of the fetus;
process the ultrasound data to identify, if present, one or more clusters of ultrasound frames, wherein for each cluster of ultrasound frames:

each ultrasound frame in the cluster of ultrasound frames belongs to a same sequence of ultrasound frames;
the ultrasound frames in the cluster of ultrasound frames are sequentially adjacent to one another;
the cluster of ultrasound frames comprises no fewer than a first predetermined number of ultrasound frames; and
the cluster of ultrasound frames comprises no more than a second predetermined number of sequentially adjacent non-heart frames;

determine, for each cluster of ultrasound frames, a quality value responsive to one or more characteristics of each heart frame in said cluster of ultrasound frames.

FIG. 1

FIG. 2

FIG. 3

400

200

Rank cluster(s) — 410

Display highest ranked cluster(s) — 420

FIG. 4

500

200

Value(s) breach threshold? — 510

Y

N

END

Recommend further imaging — 520

FIG. 5

600

200

610
Provide recommended trajectory

FIG. 6

700

200

610
Provide recommended trajectory

705
Provide complementary trajectory

710
Perform recommendation

715
Meets criteria? — Y → 720 Stop moving probe

N

725
Completed trajectory?

N

Y

730
Provide alternative recommendation

FIG. 7

# EUROPEAN SEARCH REPORT

**Application Number**

EP 24 21 7064

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/070062 A1 (PELISSIER LAURENT [CA] ET AL) 9 March 2023 (2023-03-09) * abstract * * figures 1-8 * * paragraph [0019] - paragraph [0136] * ----- | 1-15 | INV. A61B8/08 A61B8/00 |
| A | US 2023/064623 A1 (KRISHNAN KARTHIK [IN] ET AL) 2 March 2023 (2023-03-02) * abstract * * figures 1-3 * * paragraph [0006] - paragraph [0144] * ----- | 1-15 | |
| A | US 2024/206842 A1 (VAJINEPALLI PALLAVI [NL] ET AL) 27 June 2024 (2024-06-27) * abstract * * figures 1-10 * * paragraph [0005] - paragraph [0119] * ----- | 1-15 | |

**TECHNICAL FIELDS SEARCHED (IPC)**

A61B

The present search report has been drawn up for all claims

| Place of search | Date of completion of the search | Examiner |
|---|---|---|
| Munich | 16 April 2025 | Moehrs, Sascha |

EPO FORM 1503 03.82 (P04C01)

## ANNEX TO THE EUROPEAN SEARCH REPORT
## ON EUROPEAN PATENT APPLICATION NO.

EP 24 21 7064

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2025

| Patent document cited in search report | | Publication date | Patent family member(s) | | Publication date |
|---|---|---|---|---|---|
| US 2023070062 A1 | | 09-03-2023 | NONE | | |
| US 2023064623 A1 | | 02-03-2023 | CN | 115151193 A | 04-10-2022 |
| | | | EP | 4106633 A1 | 28-12-2022 |
| | | | JP | 7638291 B2 | 03-03-2025 |
| | | | JP | 2023513970 A | 04-04-2023 |
| | | | US | 2023064623 A1 | 02-03-2023 |
| | | | WO | 2021165037 A1 | 26-08-2021 |
| US 2024206842 A1 | | 27-06-2024 | CN | 116709994 A | 05-09-2023 |
| | | | EP | 4029453 A1 | 20-07-2022 |
| | | | EP | 4277532 A1 | 22-11-2023 |
| | | | JP | 2024504930 A | 02-02-2024 |
| | | | US | 2024206842 A1 | 27-06-2024 |
| | | | WO | 2022152633 A1 | 21-07-2022 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **REDMON, JOSEPH et al.** You only look once: Unified, real-time object detection. *Proceedings of the IEEE conference on computer vision and pattern recognition*, 2016
- **SUTHAHARAN, SHAN ; SHAN SUTHAHARAN**. Decision tree learning. *Machine Learning Models and Algorithms for Big Data Classification: Thinking with Examples for Effective Learning*, 2016, 247-269
- Yadt: Yet another decision tree builder. **RUGGIERI, SALVATORE.** 16th IEEE International Conference on Tools with Artificial Intelligence.. IEEE, 2004

- **JOHN C. PLATT.** Sequential minimal optimization: a fast algorithm for training support vector machines. *MSRTR: Microsoft Research 3.1*, 1998, 88-95
- Automatic confidence measure extraction for SVM outputs using neural network.. **AMINI, S. ; F. RAZZAZI ; K. NAYEBI.** 2008 International Symposium on Telecommunications.. IEEE, 2008
- **PLATT, JOHN**. Probabilistic outputs for support vector machines and comparisons to regularized likelihood methods. *Advances in large margin classifiers*, 1999, vol. 10 (3), 61-74